(19) Europäisches Patentamt / European Patent Office / Office européen des brevets

(11) **EP 2 796 091 B1**

(12) **EUROPEAN PATENT SPECIFICATION**

(45) Date of publication and mention
of the grant of the patent:
**16.03.2016 Bulletin 2016/11**

(51) Int Cl.:
*A61B 5/024* (2006.01)    *A61B 5/11* (2006.01)

(21) Application number: **14160901.6**

(22) Date of filing: **20.03.2014**

(54) **Pulse estimation device and pulse estimation program**

Pulsabschätzungsvorrichtung und Pulsabschätzungsprogramm

Dispositif et programme d'estimation d'impulsion

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.04.2013   JP 2013093069**

(43) Date of publication of application:
**29.10.2014   Bulletin 2014/44**

(73) Proprietor: **FUJITSU LIMITED
Kawasaki-shi,
Kanagawa 211-8588 (JP)**

(72) Inventor: **Kasama, Kouichirou
Kawasaki-shi, Kanagawa 211-8588 (JP)**

(74) Representative: **Lewin, David Nicholas
Haseltine Lake LLP
Lincoln House, 5th Floor
300 High Holborn
London WC1V 7JH (GB)**

(56) References cited:
**EP-A1- 2 520 222        US-A1- 2004 043 869
US-A1- 2007 249 949**

**Description**

FIELD

The embodiments discussed herein are related to pulse estimation devices and pulse estimation programs.

BACKGROUND

Conventionally, the pulse of a person during exercise is measured by a pulsimeter attached to the body of the person.

Patent Literature 1: Japanese Laid-open Patent Publication No. 2009-072417. Patent Literature 2: Japanese Laid-open Patent Publication No. 2012-232010. Patent Literature 3: European Patent Publication No. 2 520 222 A1. Patent Literature 4: US Patent Publication No. 2007/0249949 A1. Patent Literature 5: US Patent Publication No. 2004/0043869 A1

However, for a person during exercise to know his/her pulse rate, he/she needs to attach a pulsimeter to his/her body and measure his/her pulse during exercise, which is troublesome to him/her.

In one aspect, an object of the present invention is to provide a pulse estimation device and a pulse estimation program that allow a user to know his/her pulse during exercise without having to measure the pulse during exercise.

SUMMARY

According to an aspect of an embodiment, a pulse estimation device according to claim 1 is disclosed.

BRIEF DESCRIPTION OF DRAWINGS

FIG. 1 is a block diagram illustrating a functional configuration of a pulse estimation device;
FIG. 2 is a diagram illustrating an example of a data configuration of exercise intensity data;
FIG. 3 is a diagram illustrating an example of changes in pulse rate after stoppage of exercise;
FIG. 4 is a graph illustrating an example of exercise intensities for individual specific times normalized by an exercise intensity for the last specific time;
FIG. 5 is a diagram illustrating an example of normalized states of exercise intensities for individual specific times;
FIG. 6 is a diagram illustrating an example of equations for estimation of pulse rates for individual specific times;
FIG. 7 is a diagram illustrating an example of a graph showing changes in pulse rate;
FIG. 8 is a flowchart of an example of a pulse estimation process; and
FIG. 9 is a diagram of a computer executing a pulse estimation program.

DESCRIPTION OF EMBODIMENTS

Preferred embodiments of the present invention will be explained with reference to accompanying drawings. However, the present invention is not limited by these examples. The examples can be appropriately combined without causing any inconsistency between processes in the examples. In the following description, the present invention is applied to a survey system.

[a] First Embodiment Configuration of a pulse estimation device

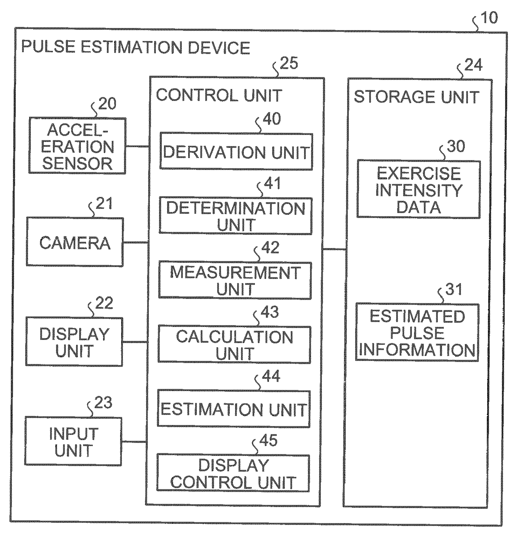

A functional configuration of a pulse estimation device 10 according to the example will be described. FIG. 1 is a block diagram illustrating a functional configuration of a pulse estimation device. The pulse estimation device 10 is a device to measure pulse after exercise to estimate pulse during exercise. The pulse estimation device 10 is a portable terminal, for example, such as a smartphone, cellular phone, PHS (personal handyphone system), or PDA (personal digital assistant). The pulse estimation device 10 may be a portable terminal device such as a notebook computer or a tablet terminal.

As illustrated in FIG. 1, the pulse estimation device 10 has an acceleration sensor 20, a camera 21, a display unit 22, an input unit 23, a storage unit 24, and a control unit 25. Besides the functional units illustrated in FIG. 1, the pulse estimation device 10 may have various functional units included in known portable terminals and terminal devices. For example, the pulse estimation device 10 may have a network interface conducting communications via a network, a carrier communication unit conducting communications via an antenna or a carrier network, a GPS (global positioning system) receiver, or the like.

The acceleration sensor 20 is a device that detects acceleration. For example, the acceleration sensor 20 is a triaxial acceleration sensor that measures accelerations in three axial directions, that is, X-axis direction, Y-axis direction, and Z-axis direction. In one mode, under control of the control unit 25, the acceleration sensor 20 detects accelerations in the three axial directions, and outputs information on the detected accelerations in the three axial directions to the control unit 25. To detect the accelerations, any arbitrary system such as a mechanical system or an optical system may be employed. In the following description, the acceleration sensor 20 is a triaxial acceleration sensor that measures accelerations in the three axial directions, but may be a G (gravitation) sensor that detects acceleration in the direction of gravitational force.

The camera 21 is an imaging device that photographs images using an imaging element such as a CCD (charge coupled device) or CMOS (complementary metal oxide semiconductor). For example, the camera 21 has three or more kinds of light-receiving elements of R (red), G (green), and B (blue) to photograph color images. As a placement example of the camera 21, when a device is equipped with a camera before shipment such as a smartphone or a cellular phone, the camera may be used as the camera 21. As another placement example of the camera 21, a digital camera or a web camera may be connected via an external terminal.

The display unit 22 is a display device that displays various kinds of information. The display unit 22 may be a display device such as an LCD (liquid crystal display) or a CRT (cathode ray tube). The display unit 22 displays various kinds of information. For example, the display unit 22 displays a graph showing changes in estimated pulse rate during exercise.

The input unit 23 is an input device that inputs various kinds of information. For example, the input unit 23 may be an input device such as a mouse or keyboard, various buttons provided in the pulse estimation device 10 or a transmission-type touch sensor provided on the display unit 22. Illustrated in FIG. 1 is an example of a functional configuration in which the display unit 22 and the input unit 23 are separated. Alternatively, the display unit 22 and the input unit 23 may be configured as an integral device such as a touch panel, for example.

The storage unit 24 is a storage device that stores various kinds of data. For example, the storage unit 24 is a storage device such as a hard disc, SSD (solid state drive), or optical disc. The storage unit 24 may be a data-rewritable semiconductor memory such as a RAM (random access memory), flash memory, or NVSRAM (non-volatile static random access memory).

The storage unit 24 stores an OS (operating system) and various programs to be executed by the control unit 25. For example, the storage unit 24 stores various programs for use in estimation of pulse. Further, the storage unit 24 stores various kinds of data for use in programs to be executed by the control unit 25. For example, the storage unit 24 stores exercise intensity data 30 and estimated pulse information 31.

The exercise intensity data 30 is data containing information on exercise intensities for individual specific times within an exercise period during which a user exercises. The exercise intensity data 30 contains, together with time information, exercise intensities derived for individual specific times within the exercise period by a derivation unit 40 described later.

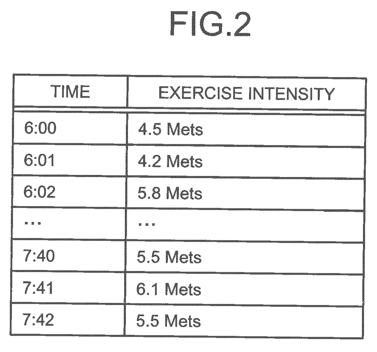

FIG. 2 is a diagram illustrating an example of a data configuration of exercise intensity data. As illustrated in FIG. 2, the exercise intensity data 30 has items of "time" and "exercise intensity." The item of time is a region for storing information indicative of time at which exercise intensity is measured. In the example, exercise intensity is measured every one minute. Stored in the item of time are times at each of which one-minute measurement of exercise intensity is started. The item of exercise intensity is a region for storing information on measured exercise intensities. In the example, the average values of activity amounts are derived as exercise intensities. Stored as exercise intensities in the item of exercise intensity are the average values of activity amounts. In the example of FIG. 2, the exercise intensity for one minute starting at time "6:00" is "4.5 Mets."

Returning to FIG. 1, the estimated pulse information 31 is data containing information on estimated pulses for individual specific times within the exercise period. The estimated pulse information 31 contains, together with time information, pulse rates for individual specific times within the exercise period that is estimated by an estimation unit 44 described later.

The control unit 25 is a device that controls the pulse estimation device 10. The control unit 25 may be an electronic circuit such as a CPU (central processing unit) or MPU (micro processing unit), or may be an integrated circuit such as an ASIC (application specific integrated circuit) or FPGA (field programmable gate array). The control unit 25 has an internal memory that stores programs and control data describing various process procedures, and executes various processes according to the programs or data. The control unit 25 operates various programs to work various systems and functions as various processing units. For example, the control unit 25 has, as processing units, the derivation unit 40, a determination unit 41, a measurement unit 42, a calculation unit 43, the estimation unit 44, and a display control unit 45.

The derivation unit 40 is a processing unit that derives exercise intensity. In the example, the derivation unit 40 derives the average value of activity amounts as exercise intensity. For example, when a predetermined operation is performed on the input unit 23 to issue an instruction for starting measurement of exercise intensity, the derivation unit 40 samples periodically acceleration at a predetermined frequency by the use of the acceleration sensor 20.

For example, when a user exercises with the pulse estimation device 10, the acceleration sensor 20 of the pulse estimation device 10 detects accelerations of the user's exercise in the three axial directions. The accelerations detected by the acceleration sensor 20 generally tend to more largely change and increase in amplitude with a higher exercise intensity.

When the user exercises, the derivation unit 40 derives the average values of activity amounts for individual specific times within the exercise period, based on the sampled accelerations. For example, when it is determined that the user is exercising, each time the acceleration sensor 20 detects accelerations in the three axial directions, the derivation unit 40 totalizes all the accelerations in the three axial directions and multiplies the totalized value by a predetermined coefficient to derive the activity amount. In addition, the derivation unit 40 derives the average value of activity amounts within a specific time period for individual specific times. For example, if it is assumed that the specific time period is one minute and the frequency of acceleration sampling is five seconds, the derivation unit 40 detects accelerations in the three axial directions by the use of the acceleration sensor 20 every five seconds. In addition, the derivation unit 40 multiplies the totalized value of accelerations in the three axial directions by a predetermined coefficient to derive activity amounts. Then, the derivation unit 40 totalizes the activity amounts derived within one minute every one minute, and divides the totalized value of activity amounts by the number of times when accelerations are sampled for one minute to derive the average value of activity amounts.

The derivation unit 40 derives the average values of activity amounts for individual specific times until the determination unit 41 described later determines stop time at which the user has stopped exercise.

The derivation unit 40 includes, together with the time information, the average values of activity amounts for individual specific times in the exercise intensity data 30. For example, the derivation unit 40 includes the start time and the average values of activity amounts for individual specific times in the exercise intensity data 30. When the average values of activity amounts are to be measured for individual specific times, the start time included may be only the first specific time.

The determination unit 41 is a processing unit that determines the stop time at which the user has stopped exercise. For example, when the totalized value of accelerations in the three axial directions detected by the acceleration sensor 20 continues to fall under a predetermined threshold value for a predetermined time period, the determination unit 41 concludes that the user has stopped exercise, and determines as stop time the first time at which the totalized value has fallen under the predetermined threshold value. The threshold value is set at a value with which, when the triaxial accelerations of exercise performed by the user carrying the pulse estimation device 10 are measured by experiment or the like, it can be regarded that the user is exercising from the accelerations, for example. For example, if it is assumed that the predetermined time period is set to 10 seconds, when the totalized value of accelerations in the three axial directions continues to fall under the predetermined threshold value for 10 seconds, the determination unit 41 determines as stop time the time at which 10 seconds start to be counted. Alternatively, the determination unit 41 may determine as the stop time the time at which the totalized value of accelerations in the three axial directions detected by the acceleration sensor 20 has fallen under the predetermined threshold value. Even if the totalized value of accelerations does not continue to fall under the predetermined threshold value for the predetermined time period, when a predetermined operation is performed on the input unit 23 to issue an instruction for starting pulse measurement, the determination unit 41 may determine as the stop time the time at which the counting is started.

The measurement unit 42 is a processing unit that measures the pulse of the user. For example, the measurement unit 42 photographs periodically images by the use of the camera 21, and measures the pulse from the periodically photographed images.

For example, the user touches the camera 21 by a finger to perform a predetermined operation on the input unit 23 to issue an instruction for starting pulse measurement.

When the predetermined operation is performed on the input unit 23 to issue an instruction for starting pulse measuring, the measurement unit 42 starts to periodically photograph images by the use of the camera 21. For example, the measurement unit 42 starts photographing of moving images in predetermined frames. Then, the measurement unit 42 detects the pulse according to subtle color changes of blood flow of fingers in the images obtained by the photographing, and measures the pulse rate from periods between peaks of the pulse. Pulse detectable portion is not limited to fingers but may be any of skin portions of the user's body. For example, the measurement unit 42 may detect the pulse and measure the pulse rate from images of the user's face periodically photographed by the camera 21. When the determination unit 41 determines that the user has stopped exercise, the measurement unit 42 may start periodical photographing of images by the camera 21.

During exercise, the pulse rate of the user changes according to exercise intensity and fluctuates at a higher level than normal. Then, when the user stops exercise, the pulse rate of the user gradually decreases to the normal level.

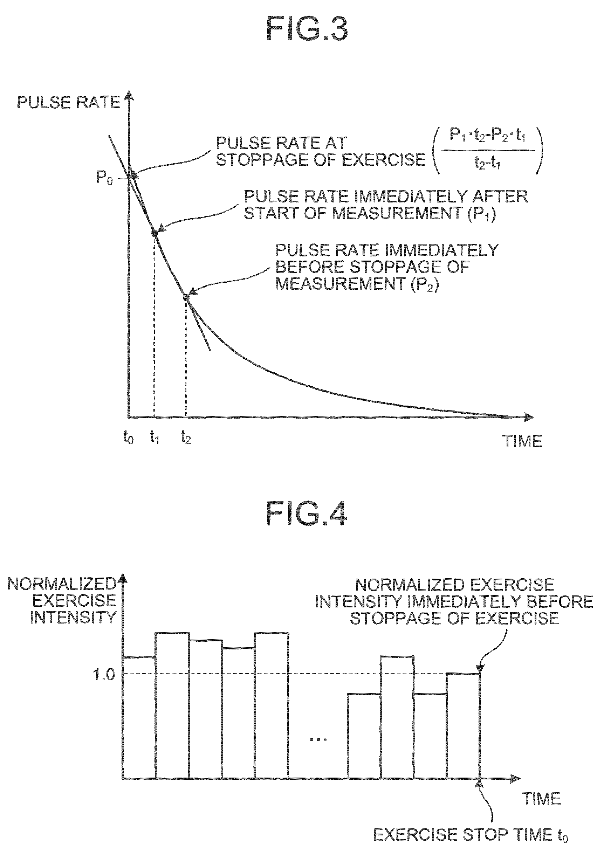

FIG. 3 is a diagram illustrating an example of changes in pulse rate after stoppage of exercise. The vertical axis in FIG. 3 represents pulse rate. The normal pulse rate is located at the origin point of the vertical axis. The horizontal axis of FIG. 3 represents time elapsed since the stop time of 0 at which the user has stopped exercise. As illustrated in FIG. 3, the pulse rate decreases gradually making a curve to the normal pulse rate, and exhibits more rapid declines at times closer to the stop time of exercise.

The calculation unit 43 is a processing unit that calculates the ratio of changes in pulse rate measured by the measurement unit 42. For example, during measurement of the user's pulse rate by the measurement unit 42 for a predetermined time period, the calculation unit 43 compares pulse rate $P_1$ measured at time $t_1$ immediately after starting of the measurement with pulse rate $P_2$ measured at time $t_2$ immediately before stoppage of the measurement, thereby to calculate the ratio of changes in the pulse rate for the predetermined time period. For example, when the predetermined time period is set to 10 seconds, the calculation unit 43 subtracts pulse rate $P_2$ at time $t_2$ immediately before stoppage of the measurement from pulse rate $P_1$ measured at time $t_1$ immediately after starting of the measurement, and divides the pulse rate resulting from the subtraction by 10 to calculate the ratio of changes in pulse rate for 10 seconds.

The estimation unit 44 is a processing unit that estimates pulse rate during exercise from the pulse rate measured by the measurement unit 42. The estimation unit 44 measures a time elapsed from the stop time determined by the determination unit 41 to the time at which the pulse has been measured by the measurement unit 42. Then, the estimation unit 44 estimates the pulse rate at stoppage of exercise, based on the elapsed time, the pulse rate measured by the measurement unit 42, and the ratio of changes calculated by the calculation unit 43. For example, the estimation unit 44 estimates the pulse rate at stoppage of exercise, based on the assumption that, after stoppage of exercise, the pulse rate decreases at the ratio of changes calculated by the calculation unit 43.

For example, the ratio of changes is calculated as $(P_1 - P_2)/(t_2 - t_1)$. If it is assumed that, after stoppage of exercise, the pulse decreases at the ratio of changes and the pulse rate at time $t_1$ is designated as $P_1$, the pulse rate $P_0$ at stoppage of exercise is determined by equation (1) as follows:

$$P_0 = (P_1 - P_2)/(t_2 - t_1) \times t_1 + P_1$$
$$= (P_1 \cdot t_1 - P_2 \cdot t_1 + P_1 \cdot t_2 - P_1 \cdot t_1)/(t_2 - t_1)$$
$$= (P_1 \cdot t_2 - P_2 \cdot t_1)/(t_2 - t_1) \tag{1}$$

The estimation unit 44 uses equation (1) to calculate pulse rate $P_0$ at stoppage of exercise from the pulse rate $P_1$ at time $t_1$ and pulse rate $P_2$ at time $t_2$.

The estimation unit 44 also estimates fluctuations in pulse rate during exercise. As described above, the pulse rate of the user during exercise changes according to exercise intensity. Thus, when exercise intensities are derived for individual specific times, fluctuations in pulse rate during exercise can be estimated from pulse rate $P_0$ at stoppage of exercise. The estimation unit 44 normalizes exercise intensities for individual specific times derived by the derivation unit 40, by the exercise intensity for the last specific time.

FIG. 4 is a graph illustrating an example of exercise intensities for individual specific times normalized by the exercise intensity for the last specific time. The vertical axis of FIG. 4 represents the exercise intensities normalized as "1.0" by the exercise intensity for the last specific time. The horizontal axis of FIG. 4 represents time during exercise. Since the pulse rate during the exercise period changes according to exercise intensity, information on the state of fluctuations in pulse rate can be obtained by the normalization.

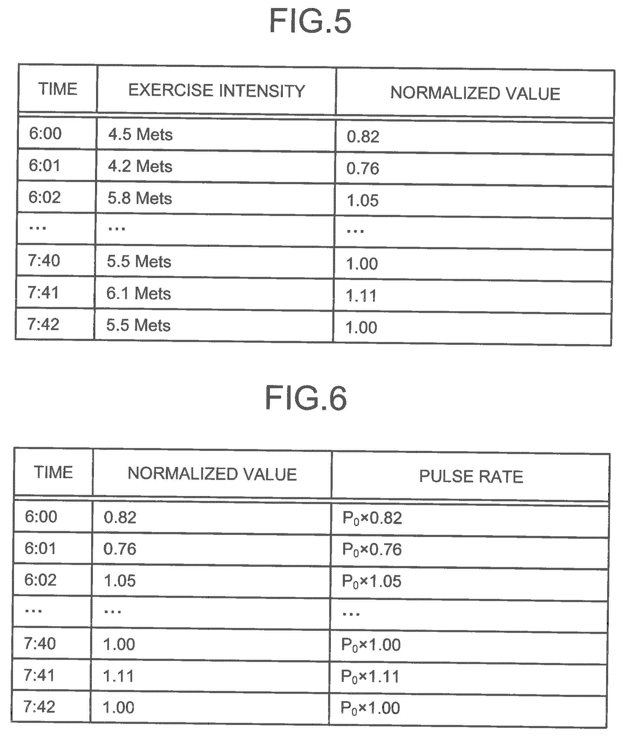

FIG. 5 is a diagram illustrating an example of normalized values of exercise intensities for individual specific times. FIG. 5 represents the normalized states of the exercise intensities for individual specific times illustrated in FIG. 2. In the example of FIG. 5, the normalized values are obtained by dividing the exercise intensities for individual specific times by exercise intensity of "5.5 Mets" for the last one minute starting from "7:42." For example, the exercise intensity is "4.5 Mets" at time "6:00," the normalized value is given as 4.5 Mets/5.5 Mets = 0.82.

The estimation unit 44 then multiplies the normalized values of exercise intensities for individual specific times by the pulse rate $P_0$ at stoppage of exercise to estimate fluctuations in pulse rate during exercise.

FIG. 6 is a diagram illustrating an example of equations for estimation of pulse rates for individual specific times. In the case of FIG. 6, the pulse rates for individual specific times are estimated using the normalized values given in FIG. 5. For example, the pulse rate at time "6:00" is estimated as a value obtained by multiplying the pulse rate $P_0$ at stoppage of exercise by the normalized value "0.82."

The estimation unit 44 includes the estimated pulse rates for individual specific times in the estimated pulse information 31.

The display control unit 45 is a processing unit that suggests to the user the estimated pulse rates for individual specific times. For example, the display control unit 45 controls the display unit 22 to display the pulse rates for individual specific times in a graph form, based on the estimated pulse information 31.

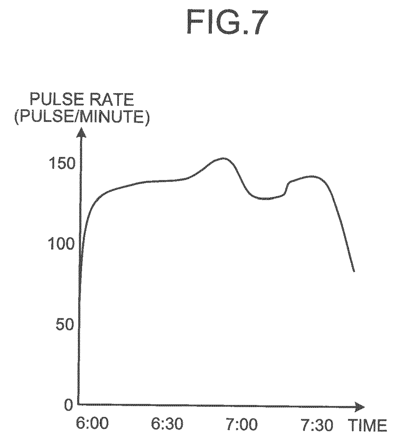

FIG. 7 is a diagram illustrating an example of a graph showing changes in pulse rate. The vertical axis of FIG.

7 represents pulse rate. The horizontal axis of FIG. 7 represents time during exercise. In the example of FIG. 7, changes in the pulse rate during exercise are given by connecting the pulse rates for individual specific times.

Accordingly, the pulse estimation device 10 can grasp the pulses during exercise.

Process flow

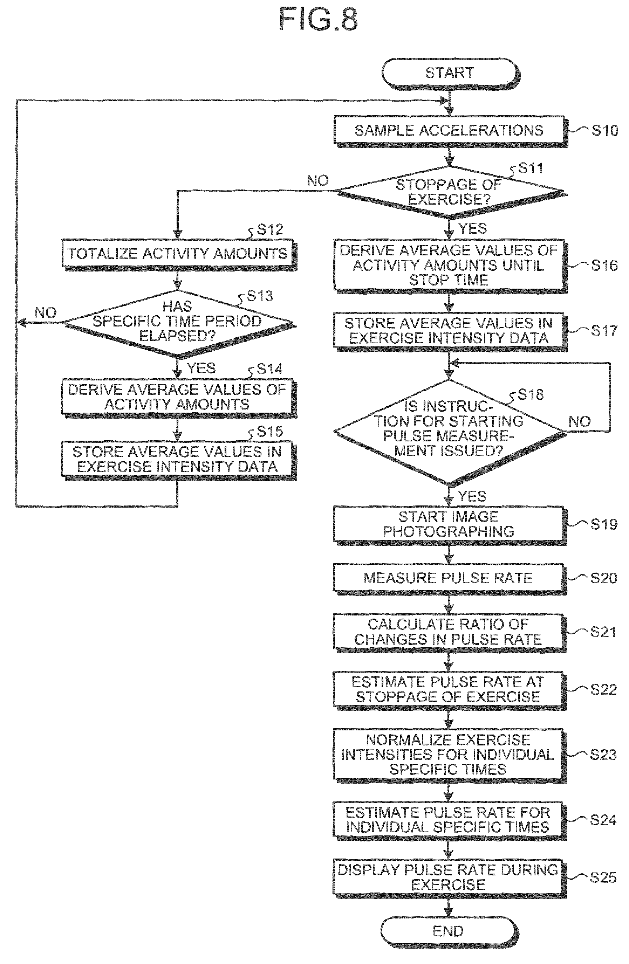

Next, a flow of a pulse estimation process for estimating the pulse rate during exercise at the pulse estimation device 10 according to the example will be described. FIG. 8 is a flow chart of an example of the pulse rate estimation process. The pulse rate estimation process is executed at a timing when the user stars exercise and performs a predetermined operation on the input unit 23 to issue an instruction for starting measurement of exercise intensity, for example.

As illustrated in FIG. 8, the derivation unit 40 samples accelerations by the use of the acceleration sensor 20 (Step S10). The determination unit 41 determines whether the user has stopped exercise, based on the accelerations detected by the acceleration sensor 20 (Step S11).

When the user has not stopped exercise (Step S11: No), the derivation unit 40 totalizes all the triaxial accelerations detected by the acceleration sensor 20, and multiplies the totalized value by a predetermined coefficient to derive the activity amounts (Step S12). The derivation unit 40 determines whether a predetermined time period has elapsed (Step S13). When the predetermined time period has not elapsed (Step S13: No), the derivation unit 40 moves to Step S10 described above. Meanwhile, when the predetermined time period has elapsed (Step S13: Yes), the derivation unit 40 derives the average value of activity amounts within a specific time period (Step S14). Then, the derivation unit 40 stores the start time and the average value of activity amounts in the specific time period in the exercise intensity data 30 (Step S15), and then moves to Step S10 described above.

Meanwhile, when the user has stopped exercise (Step S11: Yes), the derivation unit 40 derives the average value of activity amounts until the stop time within the specific time period (Step S16). The specific time period constitutes the last specific time during exercise. Then, the derivation unit 40 stores the start time and the average value of activity amounts in the last specific time in the exercise intensity data 30 (Step S17).

The measurement unit 42 determines whether a predetermined operation has been performed on the input unit 23 to issue an instruction for starting pulse measurement (Step S18). When the predetermined operation to issue an instruction for starting measurement has not been performed (Step S18: No), the measurement unit 42 moves again to Step S18 to wait for execution of the predetermined operation.

Meanwhile, when the predetermined operation for issuing an instruction for starting measurement is performed (Step S18: Yes), the measurement unit 42 starts periodical photographing of images by the use of the camera 21 (Step S19). The measurement unit 42 detects pulse based on subtle changes in blood flow of fingers from the photographed images, and measures pulse rate for a predetermined time period from a time period between the peaks of pulses (Step S20).

The calculation unit 43, during measurement of the user's pulse by the measurement unit 42 for a predetermined time period, compares pulse rate $P_1$ measured at time $t_1$ immediately after starting of the measurement with pulse rate $P_2$ measured at time $t_2$ immediately before stoppage of the measurement, thereby to calculate the ratio of changes in the pulse rate for the predetermined time period (Step S21). The estimation unit 44 estimates the pulse rate at stoppage of exercise, based on the time elapsed since the stop time, the pulse rate measured by the measurement unit 42, and the ratio of changes calculated by the calculation unit 43 (Step S22). The estimation unit 44 also normalizes exercise intensities for individual specific times during exercise by the exercise intensity for the last specific time (Step S23). The estimation unit 44 then multiplies the normalized values of exercise intensities for individual specific times by the pulse rate at stoppage of exercise to estimate pulse rates for individual specific times (Step S24). The display control unit 45 controls the display unit 22 to display the estimated pulse rates for individual specific times in a graph form (Step S25), and terminates the process.

Advantages

As described above, the pulse estimation device 10 according to the example specifies the stop time at which the user has stopped exercise. The pulse estimation device 10 also measures the pulse of the user. The pulse estimation device 10 then calculates the ratio of changes in pulse rate when the pulse of the user is measured for a predetermined time period. Then, the pulse estimation device 10 estimates the pulse rate at stoppage of exercise, based on the time elapsed from the stop time to the measurement of the pulse, the measured pulse rate, and the ratio of changes. Accordingly, the pulse estimation device 10 can grasp the pulses during exercise without having to measure the pulse during exercise.

The pulse estimation device 10 according to the example also measures pulse rate from the images photographed by the camera 21. Accordingly, even when the pulse estimation device 10 is a camera-equipped device such as a smartphone or a cellular phone, for example, it is possible to estimate pulse during exercise without having to add any new device.

The pulse estimation device 10 according to the example further has the acceleration sensor 20 to detect acceleration, and determines as the stop time the last time when the acceleration detected by the acceleration sensor 20 has fallen under a predetermined threshold value. The pulse estimation device 10 can grasp the stop time of exercise without requiring the user to input the stop time.

The pulse estimation device 10 according to the example derives exercise intensities for individual specific times during exercise. Then, the pulse estimation device 10 normalizes the derived exercise intensities for individual specific times by the exercise intensity for the last specific time, and multiplies the normalized values of the exercise intensities for individual specific times by the pulse rate at stoppage of exercise to estimate fluctuations in pulse rate during exercise. Accordingly, the pulse estimation device 10 can learn changes in pulse during exercise without having to measure pulse during exercise.

[b] Second Embodiment

The example of the device disclosed is described above. However, the technique disclosed herein can be carried out in various modes other than the foregoing example. Thus, another example included in the present invention will be described below.

For example, in the foregoing example, the pulse rate is measured from the images photographed by the camera 21, but the device disclosed herein is not limited to the foregoing example. For example, the pulse rate may be measured by a contact-type sensor capable of measuring pulses.

In the foregoing example, the average value of activity amounts is derived as exercise intensity. However, the device disclosed herein is not limited to the foregoing example. The exercise intensity may be any of values changing in conjunction with pulse rate during exercise. For example, the exercise intensity may be set as activity amount for a specific time period. In this case, when the time determined as the stop time does not constitute a marker for the last specific time, the derivation unit 40 may correct the activity amount according to the ratio until the stop time during the last specific time to derive the activity amount for the last specific time. For example, when the specific time period is one minute and the stop time is determined as a point of time at which 20 seconds of the one minute has elapsed, the derivation unit 40 triples the activity amount until the lapse of 20 seconds to derive the activity amount for the last one minute.

In the foregoing example, the ratio of changes in pulse rate for a specific time period is calculated, and the pulse rate at stoppage of exercise is estimated by straight-line approximation using the calculated ratio of changes. However, the device disclosed herein is not limited to the foregoing example. For example, data of a change model represented by the curve of FIG. 3 may be stored such that the pulse rate at stoppage of exercise can be estimated from the data of the change model. For example, data of a standard pulse change model may be stored at each exercise intensity such that the pulse rate at stoppage of exercise can be estimated using the data of the model corresponding to the average value of exercise intensities for a specific time period before stoppage of exercise. Changes in pulse after exercise varies depending on the user, and thus the data of the model may be corrected according to the user. In addition, after exercise, the pulse rate may be measured a plurality of number of times at time intervals to correct the data of the model such that the curve of changes in pulse rate passes through positions near the measured pulse rates at each of times.

In the foregoing example, the normalized values of exercise intensities for individual specific times are multiplied by the pulse rate at stoppage of exercise to estimate fluctuations in pulse rate during exercise. However, the device disclosed herein is not limited to the foregoing example. Even at constant exercise intensity, the pulse rate tends to be high when the user continues exercise for a long time. Thus, when the same exercise intensity continues during exercise, the estimation unit 44 may estimate the pulse rate at a lower level for the earlier periods of time. For example, for each of the individual specific periods of time during which the same exercise intensity continues, the estimation unit 44 may estimate the pulse rate at a lower level by a predetermined percentage than the next specific time period except for the last specific time. The predetermined percentage may be determined as a standard value through measurement of pulse rates at which the same exercise intensity continues by experiment or the like, for example.

Furthermore, the components illustrated in the drawings are functionally conceptual and do not necessarily have to be physically configured in the manner illustrated in the drawings. Specifically, the specific states of distribution and integration of the elements in the devices are not limited to those illustrated in the drawings but all or some of the elements may be functionally or physically distributed or integrated in arbitrary unit, according to various loads, use situations, and the like. For example, the processing units of the pulse estimation device 10, that is, the derivation unit 40, the determination unit 41, the measurement unit 42, the calculation unit 43, the estimation unit 44, and the display control unit 45 may be appropriately integrated. The process performed by each of the processing units may be arbitrarily divided into processes performed by a plurality of processing units. In addition, all or arbitrary ones of the functions of the processes performed by the processing units may be realized by a CPU or programs analyzed or executed by the CPU, or may be realized as wired-logic hardware.

Pulse estimation program

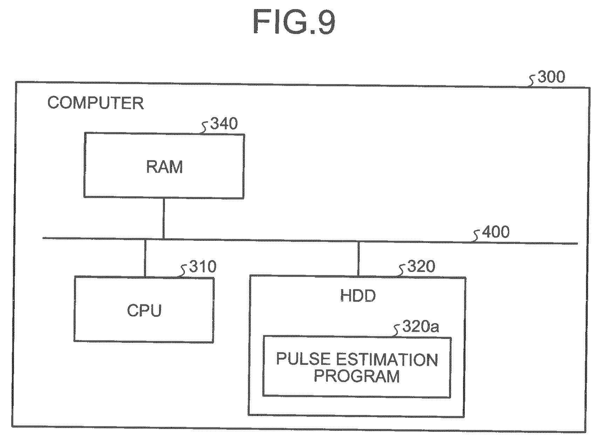

The processes described above in relation to the foregoing examples can also be realized by exercising prepared programs at a computer system such as a personal computer or a workstation. Thus, one example of a computer system executing programs with the same functions as those in the foregoing examples will be described below. FIG. 9 is a diagram of a computer executing a pulse estimation program.

As illustrated in FIG. 9, a computer 300 has a CPU (central processing unit) 310, an HDD (hard disk drive) 320, a RAM (random access memory) 340. The components 300 to 340 are connected via a bus 400.

The HDD 320 stores in advance a pulse estimation program 320a for performing the same functions as those of the derivation unit 40, the determination unit 41, the measurement unit 42, the calculation unit 43, the estimation unit 44, and the display control unit 45 of the pulse estimation device 10 described above. The pulse estimation program 320a may be appropriately separated.

The HDD 320 also stores various kinds of information. For example, the HDD 320 stores various data for use in OS and pulse estimation.

The CPU 310 reads and executes the pulse estimation program 320a from the HDD 320 to perform the same operations as those performed by the processing units in the examples. Specifically, the pulse estimation program 320a performs the same operations as those performed by the derivation unit 40, the determination unit 41, the measurement unit 42, the calculation unit 43, the estimation unit 44, and the display control unit 45.

The foregoing pulse estimation program 320a may not necessarily be stored in the HDD 320 from the beginning.

For example, the program may be stored in a "portable physical medium" such as a flexible disc (FD), CD-ROM, DVD disc, magnetic optical disc, or IC card inserted into the computer 300, for example. Then, the computer 300 may read and execute the program from the medium.

Further, the program may be stored in advance in "another computer (or server)" connected to the computer 300 via a public line, the Internet, LAN, WAN, or the like. Then, the computer 300 may read and execute the program from the computer.

According to the one aspect of the present invention, it is possible to allow a user to know his/her pulse during exercise without having to measure the pulse during exercise.

## Claims

1.   A pulse estimation device (10), comprising:

   a determination unit (41) adapted to determine a stop time at which a user has stopped exercise; and
   a measurement unit (42) adapted to measure a pulse rate of the user at a first time (t1) which occurs later than the stop time and at a second time (t2) which occurs a predetermined time period later than the first time; and
   a calculation unit (43) adapted to calculate a ratio of changes indicating a ratio of a difference between the pulse rate measured at the first time (t1) and the pulse rate measured at the second time (t2), to the predetermined time period; and
   an estimation unit (44) adapted to estimate the pulse rate at the stop time, based on a time elapsed from the stop time to the first time, the pulse rate measured at the first time and the ratio of changes.

2.   The pulse estimation device (10) according to claim 1, further comprising:

   an acceleration sensor (20) adapted to detect acceleration, wherein
   the determination unit (41) is adapted to determine as the stop time the last time at which the acceleration detected by the acceleration sensor (20) has fallen under a predetermined threshold value.

3.   The pulse estimation device (10) according to claim 1 or 2, further comprising:

   a derivation unit (40) adapted to derive, from the detected acceleration, exercise intensities respectively for individual specific times within a period of the exercise, wherein
   the estimation unit (44) is adapted to normalize the exercise intensities for individual specific times derived by the derivation unit (40), by an exercise intensity for the last specific time just before the stop time, and to multiply the normalized values of the exercise intensities for individual specific times by the estimated pulse rate at the stop time to estimate fluctuations in the pulse rate during the period of the exercise.

4. A pulse estimation program for causing a control unit of a pulse estimation device according to any of the preceding claims to execute a process comprising:

determining a stop time at which a user has stopped exercise; and
measuring a pulse rate of the user at a first time (t1) which occurs later than the stop time and at a second time (t2) which occurs a predetermined time period later than the first time; and
calculating a ratio of changes indicating a ratio of a difference between the pulse rate measured at the first time (t1) and the pulse rate measured at the second time (t2), to the predetermined time period; and
estimating a pulse rate at the stop time, based on a time elapsed from the stop time to the first time, the pulse rate measured at the first time, and the ratio of changes.


**Patentansprüche**

1. Pulsschätzvorrichtung (10), umfassend:

eine Bestimmungseinheit (41), die ausgelegt ist, eine Stoppzeit zu bestimmen, zu welcher ein Anwender eine Übung gestoppt hat; und
eine Messeinheit (42), die ausgelegt ist, eine Pulsrate des Anwenders zu einer ersten Zeit (t1), die später auftritt als die Stoppzeit, und zu einer zweiten Zeit (t2), die eine vorbestimmte Zeitperiode später als die erste Zeit auftritt, zu messen; und
eine Recheneinheit (43), die ausgelegt ist, ein Änderungsverhältnis zu berechnen, das ein Verhältnis einer Differenz zwischen der zur ersten Zeit (t1) gemessenen Pulsrate und der zur zweiten Zeit (t2) gemessenen Pulsrate zur vorbestimmten Zeitperiode anzeigt; und
eine Schätzeinheit (44), die ausgelegt ist, die Pulsrate zur Stoppzeit zu schätzen, basierend auf einer von der Stoppzeit bis zur ersten Zeit verstrichenen Zeit, der zur ersten Zeit gemessenen Pulsrate und dem Änderungsverhältnis.

2. Pulsratenschätzvorrichtung (10) gemäß Anspruch 1, weiter umfassend:

einen Beschleunigungssensor (20), der ausgelegt ist, Beschleunigung zu detektieren, wobei
die Bestimmungseinheit (41) ausgelegt ist, als die Stoppzeit die letzte Zeit zu bestimmen, zu welcher die durch den Beschleunigungssensor (20) detektierte Beschleunigung unter einen vorbestimmten Schwellenwert gefallen ist.

3. Pulsschätzvorrichtung (10) gemäß Anspruch 1 oder 2, weiter umfassend:

eine Ableitungseinheit (40), die ausgelegt ist, aus der detektierten Beschleunigung Übungsintensitäten jeweils für individuelle spezifische Zeiten innerhalb einer Periode der Übung abzuleiten, wobei
die Schätzeinheit (44) ausgelegt ist, die Übungsintensitäten für individuelle spezifische Zeiten, welche durch die Ableitungseinheit (40) abgeleitet sind, durch eine Übungsintensität für die letzte spezifizierte Zeit gerade vor der Stoppzeit zu normalisieren und die normalisierten Werte der Übungsintensitäten für individuelle spezifische Zeiten mit der geschätzten Pulsrate zur Stoppzeit zu multiplizieren, um Fluktuationen bei der Pulsrate während der Periode der Übung zu schätzen.

4. Pulsschätzprogramm, um eine Steuereinheit einer Pulsschätzvorrichtung gemäß einem der vorstehenden Ansprüche zu veranlassen, einen Prozess auszuführen, welcher umfasst:

Bestimmen einer Stoppzeit, zu welcher ein Anwender die Übung gestoppt hat; und
Messen einer Pulsrate eines Anwenders zu einer ersten Zeit t1, die später als die Stoppzeit auftritt, und zu einer zweiten Zeit t2, die eine vorbestimmte Zeitperiode später als die erste Zeit auftritt; und
Berechnen eines Änderungsverhältnisses, das ein Verhältnis einer Differenz zwischen der zur ersten Zeit (t1) gemessenen Pulsrate und der zu der zweiten Zeit (t2) gemessenen Pulsrate zur vorbestimmten Zeitperiode anzeigt; und
Schätzen einer Pulsrate zur Stoppzeit, basierend auf einer Zeit, die von der Stoppzeit bis zur ersten Zeit verstrichen ist, der durch die zur ersten Zeit gemessenen Pulsrate und dem Änderungsverhältnis.

**Revendications**

1. Dispositif d'estimation de pouls (10), comprenant :

   une unité de détermination (41) apte à déterminer un instant d'interruption auquel un utilisateur a interrompu un exercice ; et
   une unité de mesure (42) apte à mesurer une fréquence du pouls de l'utilisateur, à un premier instant (t1) qui se produit postérieurement à l'instant d'interruption et à un second instant (t2) qui se produit une période de temps prédéterminée après le premier instant ; et
   une unité de calcul (43) apte à calculer un taux de variation indiquant un rapport d'une différence entre la fréquence du pouls mesurée au premier instant (t1) et la fréquence du pouls mesurée au second instant (t2), au cours de la période de temps prédéterminée ; et
   une unité d'estimation (44) apte à estimer la fréquence du pouls à l'instant d'interruption, sur la base d'une durée écoulée depuis l'instant d'interruption jusqu'au premier instant, de la fréquence du pouls mesurée au premier instant et du taux de variation.

2. Dispositif d'estimation de pouls (10) selon la revendication 1, comprenant en outre :

   un capteur d'accélération (20) apte à détecter une accélération, dans lequel
   l'unité de détermination (41) est apte à déterminer, en tant que l'instant d'interruption, le dernier instant auquel l'accélération détectée par le capteur d'accélération (20) est passée sous une valeur de seuil prédéterminée.

3. Dispositif d'estimation de pouls (10) selon la revendication 1 ou 2, comprenant en outre :

   une unité de dérivation (40) apte à dériver, à partir de l'accélération détectée, des intensités d'exercice, respectivement, pour des temps spécifiques individuels au cours d'une période de l'exercice, dans lequel
   l'unité d'estimation (44) est apte à normaliser les intensités d'exercice, pour des temps spécifiques individuels, dérivées par l'unité de dérivation (40), par une intensité d'exercice pour le dernier instant spécifique immédiatement avant l'instant d'interruption, et à multiplier les valeurs normalisées des intensités d'exercice, pour des temps spécifiques individuels, par la fréquence du pouls estimée à l'instant d'interruption, en vue d'estimer des fluctuations de la fréquence du pouls au cours de la période de l'exercice.

4. Programme d'estimation de pouls destiné à amener une unité de commande d'un dispositif d'estimation de pouls selon l'une quelconque des revendications précédentes à exécuter un processus comprenant les étapes ci-dessous consistant à :

   déterminer un instant d'interruption auquel un utilisateur a interrompu un exercice ; et
   mesurer une fréquence du pouls de l'utilisateur, à un premier instant (t1) qui se produit postérieurement à l'instant d'interruption et à un second instant (t2) qui se produit une période de temps prédéterminée après le premier instant ; et
   calculer un taux de variation indiquant un rapport d'une différence entre la fréquence du pouls mesurée au premier instant (t1) et la fréquence du pouls mesurée au second instant (t2), au cours de la période de temps prédéterminée ; et
   estimer la fréquence du pouls à l'instant d'interruption, sur la base d'une durée écoulée depuis l'instant d'interruption jusqu'au premier instant, de la fréquence du pouls mesurée au premier instant et du taux de variation.

PULSE ESTIMATION DEVICE 10

CONTROL UNIT 25

STORAGE UNIT 24

ACCEL-ERATION SENSOR 20

CAMERA 21

DISPLAY UNIT 22

INPUT UNIT 23

DERIVATION UNIT 40

DETERMINATION UNIT 41

MEASUREMENT UNIT 42

CALCULATION UNIT 43

ESTIMATION UNIT 44

DISPLAY CONTROL UNIT 45

EXERCISE INTENSITY DATA 30

ESTIMATED PULSE INFORMATION 31

# FIG.2

| TIME | EXERCISE INTENSITY |
|------|--------------------|
| 6:00 | 4.5 Mets |
| 6:01 | 4.2 Mets |
| 6:02 | 5.8 Mets |
| . . . | . . . |
| 7:40 | 5.5 Mets |
| 7:41 | 6.1 Mets |
| 7:42 | 5.5 Mets |

# FIG.3

PULSE RATE

PULSE RATE AT STOPPAGE OF EXERCISE $\left( \dfrac{P_1 \cdot t_2 - P_2 \cdot t_1}{t_2 - t_1} \right)$

PULSE RATE IMMEDIATELY AFTER START OF MEASUREMENT ($P_1$)

PULSE RATE IMMEDIATELY BEFORE STOPPAGE OF MEASUREMENT ($P_2$)

$P_0$

$t_0$  $t_1$  $t_2$

TIME

# FIG.4

NORMALIZED EXERCISE INTENSITY

NORMALIZED EXERCISE INTENSITY IMMEDIATELY BEFORE STOPPAGE OF EXERCISE

1.0

...

TIME

EXERCISE STOP TIME $t_0$

# FIG.5

| TIME | EXERCISE INTENSITY | NORMALIZED VALUE |
|------|--------------------|--------------------|
| 6:00 | 4.5 Mets | 0.82 |
| 6:01 | 4.2 Mets | 0.76 |
| 6:02 | 5.8 Mets | 1.05 |
| ... | ... | ... |
| 7:40 | 5.5 Mets | 1.00 |
| 7:41 | 6.1 Mets | 1.11 |
| 7:42 | 5.5 Mets | 1.00 |

# FIG.6

| TIME | NORMALIZED VALUE | PULSE RATE |
|------|------------------|------------|
| 6:00 | 0.82 | $P_0 \times 0.82$ |
| 6:01 | 0.76 | $P_0 \times 0.76$ |
| 6:02 | 1.05 | $P_0 \times 1.05$ |
| ... | ... | ... |
| 7:40 | 1.00 | $P_0 \times 1.00$ |
| 7:41 | 1.11 | $P_0 \times 1.11$ |
| 7:42 | 1.00 | $P_0 \times 1.00$ |

# FIG.7

PULSE RATE
(PULSE/MINUTE)

150

100

50

0

6:00    6:30    7:00    7:30   TIME

# FIG.8

```
                                    ┌──────────┐
                                    │  START   │
                                    └────┬─────┘
                                         ↓
                              ┌──────────────────────┐
                              │ SAMPLE ACCELERATIONS  │  S10
                              └──────────┬───────────┘
                                         ↓
                            ┌─────────────────────┐
              NO            │    STOPPAGE OF      │  S11
       ┌──────────────────◄ │     EXERCISE?       │
       │                    └──────────┬──────────┘
       │                               ↓ YES
       ↓                    ┌───────────────────────────┐
┌─────────────────────┐    │ DERIVE AVERAGE VALUES OF   │  S16
│ TOTALIZE ACTIVITY   │    │ ACTIVITY AMOUNTS UNTIL     │
│ AMOUNTS             │    │ STOP TIME                  │
│  S12                │    └────────────┬──────────────┘
└──────────┬──────────┘                 ↓
           ↓                 ┌───────────────────────────┐
   ┌──────────────┐          │ STORE AVERAGE VALUES IN    │  S17
NO │   HAS        │          │ EXERCISE INTENSITY DATA    │
◄──│ SPECIFIC TIME│  S13     └────────────┬──────────────┘
   │ PERIOD       │                       ↓
   │ ELAPSED?     │          ┌───────────────────────────┐
   └──────┬───────┘     NO   │  IS INSTRUC-              │  S18
          ↓ YES    S14  ┌───◄│ TION FOR STARTING         │
┌─────────────────────┐ │    │ PULSE MEASURE-            │
│ DERIVE AVERAGE      │ │    │ MENT ISSUED?             │
│ VALUES OF ACTIVITY  │ │    └────────────┬─────────────┘
│ AMOUNTS             │ │                 ↓ YES
└──────────┬──────────┘ │    ┌───────────────────────────┐
           ↓       S15  │    │ START IMAGE               │  S19
┌─────────────────────┐ │    │ PHOTOGRAPHING             │
│ STORE AVERAGE       │ │    └────────────┬─────────────┘
│ VALUES IN EXERCISE  │ │                 ↓
│ INTENSITY DATA      │ │    ┌───────────────────────────┐
└─────────────────────┘ │    │ MEASURE PULSE RATE        │  S20
                        │    └────────────┬─────────────┘
```

Step S10: SAMPLE ACCELERATIONS

Step S11: STOPPAGE OF EXERCISE?

Step S12: TOTALIZE ACTIVITY AMOUNTS

Step S13: HAS SPECIFIC TIME PERIOD ELAPSED?

Step S14: DERIVE AVERAGE VALUES OF ACTIVITY AMOUNTS

Step S15: STORE AVERAGE VALUES IN EXERCISE INTENSITY DATA

Step S16: DERIVE AVERAGE VALUES OF ACTIVITY AMOUNTS UNTIL STOP TIME

Step S17: STORE AVERAGE VALUES IN EXERCISE INTENSITY DATA

Step S18: IS INSTRUCTION FOR STARTING PULSE MEASUREMENT ISSUED?

Step S19: START IMAGE PHOTOGRAPHING

Step S20: MEASURE PULSE RATE

Step S21: CALCULATE RATIO OF CHANGES IN PULSE RATE

Step S22: ESTIMATE PULSE RATE AT STOPPAGE OF EXERCISE

Step S23: NORMALIZE EXERCISE INTENSITIES FOR INDIVIDUAL SPECIFIC TIMES

Step S24: ESTIMATE PULSE RATE FOR INDIVIDUAL SPECIFIC TIMES

Step S25: DISPLAY PULSE RATE DURING EXERCISE

END

# FIG.9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009072417 A
- JP 2012232010 A
- EP 2520222 A1
- US 20070249949 A1
- US 20040043869 A1